(19) Europäisches Patentamt
European Patent Office
Office européen des brevets

(11) Veröffentlichungsnummer: **0 029 617**
**B1**

# (12) EUROPÄISCHE PATENTSCHRIFT

(45) Veröffentlichungstag der Patentschrift: **12.09.84**

(51) Int. Cl.[3]: **C 07 D 295/02**, C 07 D 295/04, C 07 D 211/14, C 07 D 203/08, A 01 N 43/34

(21) Anmeldenummer: **80200923.3**

(22) Anmeldetag: **01.10.80**

(54) **Verwendung tertiärer heterocyclischer Amine als Potenzierungsmittel für Insektizide; Cyclo-Amin-Derivate.**

(30) Priorität: **27.11.79 DE 2947648**

(43) Veröffentlichungstag der Anmeldung: **03.06.81 Patentblatt 81/22**

(45) Bekanntmachung des Hinweises auf die Patenterteilung: **12.09.84 Patentblatt 84/37**

(84) Benannte Vertragsstaaten: **AT BE CH DE FR GB IT LI LU NL SE**

(56) Entgegenhaltungen:
**EP - A - 0 006 992**
**EP - A - 0 016 731**
**DE - A - 2 459 129**
**DE - B - 1 032 023**
**FR - A - 1 360 329**
**FR - A - 1 604 269**
**FR - A - 2 111 960**
**FR - A - 2 308 364**
**FR - A - 2 357 177**
**US - A - 3 297 703**
**US - A - 4 175 187**

**CHEMICAL ABSTRACTS, Band 69, Nr. 3, 11. Juli 1968, Seite 1009, Nr. 10543t Columbus, Ohio, U.S.A. T. KURIHARA et al.: "Ulcer remedies"**
**CHEMISTRY AND INDUSTRY, Nr. 5, 5. März 1977, Seite**

(73) Patentinhaber: **C.F. Spiess & Sohn GmbH & Co. Chemische Fabrik, D-6719 Kleinkarlbach (DE)**

(72) Erfinder: **Himmelreich, Rolf G., Uhlandstrasse 9, D-6718 Grünstadt (DE)**
Erfinder: **Spiess, Wolfram, Dr. Dipl.-Chem., Heinrich-Becker-Strasse 16, D-6718 Grünstadt (DE)**

(74) Vertreter: **Eggert, Hans-Gunther, Dr., Räderscheidtstrasse 1, D-5000 Köln 41 (DE)**

(56) Entgegenhaltungen: (Fortsetzung)
**202 M. TANAKA et al.: "Base-catalysed isomerisation of N-(3,7-dimethyl-octa-2,6-dienyl)dialkylamine to N-(3,7-dimethylocta-1,3-dienyl)dialkylamine: dihydrocitral from isoprene"**
**BULLETIN OF THE CHEMICAL SOCIETY OF JAPAN, Band 46, Nr. 1, Januar 1973, Seiten 222-225 K. TAKABE et al.: "Syntheses of isoprenoids by telomerizations. VIII. Anionic telomerizations of isoprene with secondary amines"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 617 B1

ACTORUM AG

**Beschreibung**

Die vorliegende Erfindung betrifft die Verwendung tertiärer, heterocyclischer Amine der allgemeinen Formel (I)

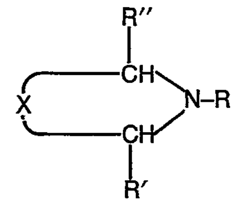

I

worin

R ein aliphatischer Rest mit 1 bis 22 Kohlenstoffatomen, der durch Aryl-, Hydroxyl-, Alkoxy- und/oder Carboxylestergruppen oder durch den Rest des heterocyclischen Amins der Formel (I) (ohne R) substituiert sein kann und der ausserdem durch Äther- oder Estergruppen unterbrochen sein kann, oder Piperonyl

R′ und R″ unabhängig voneinander jeweils H, $CH_3$ oder $C_2H_5$ und

X die Gruppe $-(CH_2)n-$, worin n eine ganze Zahl von 0 bis 5 ist, oder die Gruppe $-CH_2-O-CH_2-$ bedeuten, als Potenzierungsmittel für insektizide Pyrethroide und/oder Carbamate und/oder Phosphorsäurederivate.

In CA 69 (1968), Referat 10543 t ist die Synthese von N-Geranyl-pepiridinhydrochloriden, Geranyl-Essigsäureestern, Geranyläthern und anderen Geranylderivaten beschrieben. Die Produkte sollen eine antispasmolytische Wirkung haben.

M. Tanaka und Go Hata beschreiben in Chemistry and Industry 5 (1977), Seite 202 die durch Basen katalysierte Isomerisierung von N-3,7-Dimethylocta-2,6-dienyl-dialkylaminen zu N-(3,7-Dimethylocta-1,3-dienyl)dialkylaminen, insbesondere die Herstellung von Dihydrozitral aus entsprechenden Isoprenderivaten als eine einfache Methode zum Herstellen von Aldehyden aus Alkyl-1,3-Dienen.

In Bull. Chem. Soc. Japan 46, 1973, Seiten 222 bis 225 ist die Synthese von Isoprenoidderivaten durch Telomerisation beschrieben, insbesondere die stereoselektive Wirkung von Telomeren bei der Telomerisation von Isoprenderivaten mit sekundären Aminen.

In FR-A-1 360 329 sind tertiäre Amine als Fungizide für die Landwirtschaft beschrieben. Dabei kann es sich um heterocyclische Verbindungen mit 3 bis 7 Ringen handeln.

In FR-A-2 357 177 sind Pyrethroide beschrieben, die pestizide Wirkung aufweisen. Diese können natürlichen Ursprungs oder synthetisiert sein. Im Falle von Verbindungen natürlichen Ursprungs handelt es sich um solche, die aus Pfeffer, Eukalyptus oder Geraniol gewonnen werden.

Diese beanspruchten Amine, von denen überraschenderweise einige selbst eine insektizide Wirkung haben, zeigen im Zusammenwirken mit insektiziden Phosphorsäurederivaten und/oder Carbamaten und/oder Pyrethroiden eine deutliche Wirkungssteigerung. Die heterocyclischen Amine haben nur eine geringe Toxizität auf Warmblüter. Sie sind daher sicherer zu handhaben und weniger gefährlich für Mensch und Nutztier als die meisten der bisher verwendeten Insektizide.

Wegen ihrer guten synergistischen Eigenschaften können diese insektiziden Mischungen bei gleicher Wirkung in wesentlich geringeren Aufwandmengen eingesetzt werden, als bisher üblich, was eine wesentlich geringere ökologische Belastung bedeutet.

Während heterocyclische Amine, die am Stickstoffatom des heterocyclischen Rings nicht substituiert sind, im Kombination mit Phosphorsäureestern, Carbamaten oder Pyrethroiden unwirksam sind, erwiesen sich Verbindungen, in denen der Wasserstoff der –NH-Gruppe durch eine Gruppe R ersetzt ist, als ausgesprochen wirksam. Überraschenderweise kann R für eine Vielzahl verschiedener Gruppierungen stehen. So kann R für aliphatische unverzweigte Reste, verzweigte aliphatische Reste, olefinische Reste, die einfach oder mehrfach ungesättigt, unverzweigt oder verzweigt sind, mit 1 bis 22 Kohlenstoffatomen stehen. Der Rest R kann Hydroxylgruppen enthalten, wie beispielsweise in der Gruppierung $-CH_2-CH_2OH$ oder $-CH_2-CHOH-CH_2OH$. Der Rest R kann auch für gemischte aliphatisch-aromatische Verbindungen stehen. Der Rest R kann auch eine oder mehrere Carboxyl- oder Estergruppen enthalten. Der Rest R kann ausserdem als Substituenten das gleiche oder ein anderes tertiäres, heterocyclisches Amin der allgemeinen Formel (I) enthalten, in der lediglich R weggelassen wird, wobei eine Diaminverbindung erhalten wird.

Bevorzugt als Substituenten sind die folgenden Reste:

Pentyl, Hexyl, Heptyl, Octyl, Nonyl,
Decyl, Undecyl, Dodecyl, Tridecyl, Tetradecyl,
Hexadecyl, Docosyl, Linalyl, Citronellyl,
Farnesyl, 2,6-Dimethyloctadienyl,
3,7-Dimethyloctadien-(2,6)-yl, Methyl-hepten-(3)-yl
3,7,11-Trimethyl-1,6,10-dodecatrienyl,
Naphthylmethyl, Cinnamyl, Benzyl, Benzal
(z.B. diaminsubstituiert), Diphenylmethyl,
Phenyl-ethyl-(2)-, 1-Phenyl-1,3-butadienyl,
3-Phenylpropen-(2)-yl, Propargyl, Allyl,
Propinyl, 4-Chlorbenzyl,
α-Butyltriäthylen-triäther(ω),
1-Phenoxypropyl-(3), 2′-Butoxy-diethyläther-(2)-,
1′-Ethoxyäthyl-(2)-, 2-Phenoxyethyl-(1)-,
3-Phenoxy-propyl-(3)-, 2-Benzyloxy-ethyl-1,
Propandiol-(2,3), Hydroxyäthyl,
2,2-Bis-(hydroxymethyl)-propan-3-ol-1-yl,
Ethyldibutylacetal, Methylheptanal,
Phenylessigsäureethylester- und der
Bernsteinsäurediethylester-Rest,
Dimethylcarbamoyl und Piperonyl.

Wenn der Rest R ein weiteres tertiäres heterocyclisches Amin enthält, besteht er vorzugsweise aus einem Alkylrest mit 1 bis 22 Kohlenstoffatomen, vorzugsweise einem Alkylrest mit 3 bis 10 Kohlenstoffatomen, wie z.B. Propyl, Butyl, Pentyl, Hexyl, Heptyl, Octyl, Nonyl, Dekyl.

Als heterocyclische stickstoffhaltige Ringverbindung sind Aziridin, Pyrrolidin, Piperidin, Hexamethylenimin sowie die substituierten Heterocyclen 2-Methylpyrrolidin, 2,5-Dimethylpyrrolidin, 2-Methylpiperidin, 2-Ethylpiperidin, und Morpholin bevorzugt.

Die erfindungsgemässen tertiären, heterocyclischen Amine werden in bekannter Weise hergestellt, indem das Halogenid des Restes der am Stickstoffatom zu substituierenden Gruppierung, am zweckmässigsten der Chlor- oder Bromverbindungen, mit dem heterocyclischen Amin umgesetzt wird. Dabei kann man in vielen Fällen mit einem Molverhältnis von 1:1 arbeiten und erhält das Hydrohalogenid der gewünschten Verbindung, aus dem durch Reaktion mit Lauge das tertiäre Amin gewonnen wird. In einigen Fällen ist es ratsam, 2 Mol Amin mit 1 Mol Halogenverbindung zur Reaktion zu bringen. Man kann die dabei anfallenden heterocyclischen Amin-Hydrohalogenidverbindungen anschliessend wieder ins Amin überführen. Anstelle eines zweiten Mols heterocyclischen Amins kann man in vielen Fällen auch ein preiswertes tertiäres Amin als Hilfsamin einsetzen, beispielsweise Triäthylamin. Die Reaktion kann mit oder ohne Lösungsmittel ausgeführt werden. In einigen Fällen muss stark gekühlt werden, häufiger ist es jedoch notwendig, die Mischung eine Zeitlang zu erwärmen, wobei in einigen Fällen bis 160 °C erhitzt werden muss, um die Umsetzung zu erreichen. Man kann die erfindungsgemässen tertiären, heterocyclischen Amine aber auch durch Ringbildung, z.B. aus Derivaten von Diolen und primären Aminen, erhalten. Zum Beispiel werden 1-Hexyl-Piperidin und analoge Verbindungen aus Pentan-(1,5)-bis-benzolsulfonsäureester und Hexylamin oder anderen primären Aminen erhalten.

Die Wirksamkeit der tertiären-heterocyclischen Amine wird durch eine Vielzahl von Beispielen deutlich gemacht. Als Versuchstiere wurden dabei Kornkäfer (sitophilus granarius L.) und Taufliegen (drosophila melanogaster) eingesetzt.

Die erfindungsgemässen tertiären, heterocyclischen Amine geben im Gemisch mit Phosphorsäureester und/oder Carbamaten und/oder Pyrethroiden eine starke insektizide Wirkungssteigerung, abgesehen davon, dass sie zum Teil selbst insektizide Eigenschaften haben. Dieser Synergismus wurde dadurch nachgewiesen, dass die Insektizide in den nachfolgenden Versuchen in Konzentrationen angewandt wurden, bei denen sich gerade erste insektizide Wirkungen zeigten. Durch Zusatz der erfindungsgemässen Amine wird die Wirkung der Insektizide wesentlich gesteigert.

Einige der erfindungsgemässen Amine zeigen selbst eine starke, insektizide Wirkung, so z.B. die Decyl- und Undecylverbindungen des Piperidins und des Pyrrolidins, aber auch die Phenylessigsäure-Ethylesterderivate dieser beiden Amine. Auch diese Verbindungen zeigen im Gemisch mit den bekannten Insektiziden eine synergistische Wirkung, die allerdings erst bei entsprechend starker Verdünnung nachweisbar ist.

Als Insektizide wurden bei diesen Versuchen Cypermethrin und Permethrin als Vertreter der Pyrethroide, Dimethoat als Vertreter der Phosphorester und Dimetilan als Vertreter der Carbamate eingesetzt.

Cypermethrin

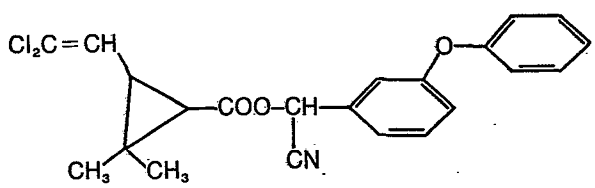

Permethrin

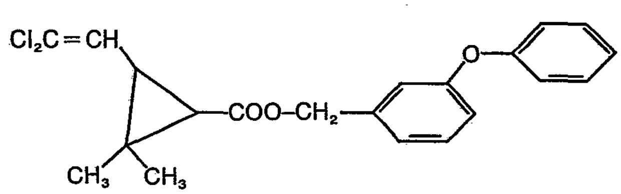

Dimethoat

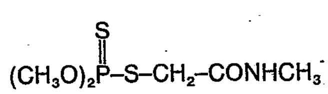

Dimetilan

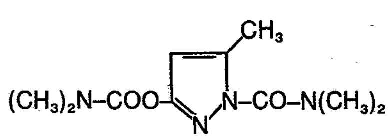

Das Mengenverhältnis von eingesetztem Insektizid zu tertiärem, heterocyclischem Amin kann zwischen 1:0,2 und 1:100 liegen, wobei sich ein Verhältnis zwischen 1:5 und 1:10 als besonders günstig erwies. Bei gleicher Wirksamkeit kann das Insektizid in vielen Fällen mit 1/10 bis 1/25 der in der Praxis üblichen Aufwandmenge eingesetzt werden.

Beispiel 1

Die insektiziden Wirkungen von tertiären heterocyclischen Aminen und die synergistischen Eigenschaften mit dem Phosphorsäureester Dimethoat und dem Carbamat Dimetilan wurden an Drosophila melanogaster getestet.

Die Versuche wurden in Petrischalen von 10 cm Durchmesser ausgeführt. Es wurden zunächst die gerade noch wirksamen Konzentrationen von Dimethoat und Dimetilan bestimmt. Für Dimethoat wurde 0,005% und für Dimetilan 0,0001% als günstige Versuchskonzentration ermittelt. Die Aminkomponente wurde mit 1 ml 0,05%iger Lösung + 1 ml Aceton auf insektizide Wirkung getestet. Zur Bestimmung des Synergismus wurde 1 ml 0,05%ige Aminlösung + 1 ml 0,005%ige Dimethoatlösung bzw. + 1 ml 0,0001%ige Dimetilanlösung pro Petrischale eingesetzt. Alle Verdünnungen wurden mit Aceton als Lösungsmittel hergestellt.

In den Petrischalen wurden die Lösungen am Boden gut verteilt und 30 min lang antrocknen lassen. In jede Petrischale wurden 20 mit Kohlendioxid betäubte Drosophila eingezählt. Alle 5 min, insgesamt 12mal, wurde die Anzahl der toten Drosophila ausgezählt und notiert. Die Zahlen wurden aufsummiert und im Verhältnis zur möglichen Zahl an toten Drosophila in % angegeben, wobei der Tod aller Drosophila innerhalb der ersten 5 min 100% Wirkung entspricht. Jeder Versuch wurde mit 3 Wiederholungen durchgeführt. In Tabelle 1 sind die Mittelwerte der Ergebnisse zusammengestellt.

Zur Ermittlung des synergistischen Faktors wurden die Einzelwerte pro Zeiteinheit durch die für Dimethoat bzw. Dimetilan für die jeweilige Zeiteinheit gefundenen Werte dividiert. Die Werte für jede Zeiteinheit wurden summiert und durch die Anzahl der Zeiteinheiten dividiert. Die Versuche wurden bei sonst stets gleichbleibenden Bedingungen bei Temperaturen von 20–22 °C ausgeführt. Die Kontrollversuche zeigten bei Versuchsabschluss stets 0% Mortalität. Hat die Substanz selbst eine insektizide Wirkung, so wurden die Wirkungsfaktoren für Dimethoat und Dimetilan nach Abzug des Substanzwertes berechnet und in der Tabelle aufgeführt.

Tabelle 1

Tests an Drosophila melanogaster
Wirksamkeit von Dimethoat: 8,6%
Wirksamkeit von Dimetilan: 2,5%

| Name der Substanz | Wirksamkeit in % | | | Faktor der Wirkungssteigerung | |
|---|---|---|---|---|---|
| | Substanz allein | Substanz + Dimethoat | Substanz + Dimetilan | bei Dimethoat | bei Dimetilan |
| N-1-Linalyl-aziridin | 0 | 21,7 | 0,42 | 2,67 | – |
| N-(3-Cyclohexyl-propen-(2))-pyrrolidin | 0 | 22,5 | 0 | 2,86 | – |
| N-(α-Butyl-triethylen-triether-(ω))-pyrrolidin | 0 | 23,3 | 1,7 | 2,97 | – |
| 1-Cinnamyl-pyrrolidin | 0 | 5,8 | 55,4 | – | 22,2 |
| N-1-Decyl-pyrrolidin | 2,9 | 17,1 | 4,8 | 1,65 | – |
| N-1-Tridecyl-piperidin | 0 | 54,6 | 2,3 | 6,35 | – |
| N-(1-Phenoxi-propyl-(3))-piperidin | 0 | 8,8 | 12,1 | – | 4,8 |
| Phenyl-N-piperidino-essigsäureethylester | 0 | 22,2 | 2,7 | 2,57 | – |
| N-Piperidino-bernsteinsäure-di-ethylester | 0 | 38,8 | 2,3 | 4,51 | – |
| 1-n-Nonyl-2-ethyl-piperidin | 5,8 | 24,6 | 7,9 | 2,19 | – |
| 1,8-N,N-Dimorpholino-octan | 1,3 | 23,8 | 10,4 | 2,62 | 3,64 |
| N-(2′-Butoxi-diethylether-(2))-morpholin | 1,7 | 12,9 | 3,5 | 1,30 | – |
| 2-N-Morpholino-ethyl-dibutyl-acetal | 0,4 | 17,9 | 3,3 | 2,03 | 1,16 |
| N-1-Linalyl-morpholin | 11,7 | 30,8 | 20,0 | 2,22 | 3,32 |
| N-1-Tetradecyl-morpholin | 0 | 57,1 | 2,5 | 6,64 | – |
| N-Cinnamyl-piperidin | 66,3 | 77,1 | 82,5 | 1,25 | 6,48 |
| N-Piperonyl-piperidin | 51,7 | 65,0 | 52,1 | 1,55 | – |

Tabelle 1 (Fortsetzung)

| Name der Substanz | Wirksamkeit in % | | | Faktor der Wirkungssteigerung | |
|---|---|---|---|---|---|
| | Substanz allein | Substanz + Dimethoat | Substanz + Dimetilan | bei Dimethoat | bei Dimetilan |
| N-Decyl-2-methyl-piperidin | 61,3 | 66,7 | 77,9 | – | 2,87 |
| N-Decyl-hexamethylenimin | 49,2 | 65,0 | 68,8 | 1,84 | 7,84 |
| 6-N-Pyrrolidino-undecan | 39,6 | 54,2 | 75,8 | 1,70 | 14,48 |
| N-Piperonyl-pyrrolidin | 28,8 | 60,0 | 58,3 | 3,63 | 11,80 |
| N-1-Decyl-2-ethyl-piperidin | 35,0 | 55,8 | 56,3 | 2,42 | 8,52 |

In allen Tabellen bedeutet «–» oder «0», dass die gewählte Konzentration die Wirksamkeit oder den Synergismus nicht deutlich erkennen liess.

Beispiel 2

Im Beispiel 1 war das Verhältnis von Insektizid und erfindungsgemässem Amin 1 : 10 in den Gemischen mit Dimethoat. In der Tabelle 2 sind Verhältnisse von 1 : 1, 1 : 2 und 1 : 0,2 bei Versuchen mit Dimethoat aufgeführt, dabei wurde das Dimethoat immer in einer Konzentration von 0,005% eingesetzt. Die Tabelle zeigt, dass auch bei diesen Verhältnissen noch eine gute synergistische Wirkung vorhanden ist, und es keineswegs einer grossen Überdosierung des Amins bedarf.

Tabelle 2

Test an Drosophila melanogaster mit Dimethoat im Gemisch mit wechselnden Konzentrationen tertiärer heterocyclischer Amine.
(Wirksamkeit von Dimethoat bei 0,005% : 8,6%)

| Name der Substanz | Konzentration % | Verhältnis Dimethoat : Amin | Substanz allein | Substanz + Dimethoat | Faktor der Wirkungssteigerung |
|---|---|---|---|---|---|
| N-1-Tridecyl-piperidin | 0,05 | 1:10 | 0 | 54,6 | 6,35 |
| N-1-Tridecyl-piperidin | 0,01 | 1:2 | 0 | 42,9 | 4,99 |
| N-Citronellyl-piperidin | 0,005 | 1:1 | 0 | 32,9 | 3,83 |
| N-Citronellyl-piperidin | 0,001 | 1:0,2 | 0 | 30,4 | 3,53 |
| Phenyl-N-piperidino-essigsäure-ethylester | 0,05 | 1:10 | 0 | 22,1 | 2,57 |
| Phenyl-N-piperidino-essigsäure-ethylester | 0,01 | 1:2 | 0 | 59,2 | 6,88 |
| N-1-Decyl-piperidin | 0,005 | 1:1 | 0 | 45,4 | 5,28 |
| N-Piperonyl-piperidin | 0,05 | 1:10 | 28,8 | 60,0 | 3,63 |
| N-Piperonyl-piperidin | 0,005 | 1:1 | 0 | 26,3 | 3,06 |

Beispiel 3

Test von tertiären, heterocyclischen Aminen allein und im Gemisch mit Pyrethroiden an Kornkäfern (sitophilus granarius L).

In Petrischalen mit 15 cm Durchmesser mit talcumbepuderten Seitenwänden, um das Entfliehen der Kornkäfer zu verhindern, wurden jeweils 2 ml der Acetonlösungen einpipettiert und durch vorsichtiges Schwenken der Schalen gleichmässig auf dem Boden verteilt. Nach dem Abdampfen des Acetons und dem Antrocknen der Beläge wurden in jede Schale 25 Kornkäfer eingezählt. Nach einer Stunde wurde jeweils ein 12,5 cm Rundfilter in die Schalen eingelegt, um den Käfern das Befreien aus der Rückenlage zu ermöglichen, was auf der glatten Glasplatte nicht möglich ist. Die lichtscheuen Käfer verkrochen sich mit Vorliebe unter die Filter und bleiben so ständig mit den Wirkstoffbelägen in Kontakt. Alle Versuche liefen mit 3 Wiederholungen. Nach 3 h wurden die toten Käfer ausgezählt.

In der Tabelle 3 ist jeweils der Durchschnitt der Summe der 4 Einzelwerte angegeben. 25 tote Kornkäfer entsprechen dabei einer Wirkung von 100%. Die Temperaturen betrugen während der Tests im Durchschnitt 19–21 °C. Der synergistische Faktor berechnet sich analog wie in Beispiel 1 angegeben.

Die zu prüfenden tertiären, heterocyclischen Amine wurden mit 1 mg Wirkstoff pro ml als Acetonlösungen angesetzt. Sie wurden im Verhältnis 1 : 1 mit Cypermethrin bzw. Permethrin, die mit 0,01 mg Wirkstoff pro ml als Acetonlösung vorlagen, gemischt. Die Einzelkomponenten wurden im Verhältnis 1 : 1 mit Aceton gemischt.

In der Tabelle 3 sind die Wirkstoffe nach der Art des heterocyclischen Amins geordnet.

Auch bei diesen Versuchen zeigten die Kontrollversuche stets bei Versuchsende eine Mortalität der Kornkäfer von 0%.

Tabelle 3

Tests an sitophilus granarius L.
Wirksamkeit von Cypermethrin: 31,5%
Wirksamkeit von Permethrin: 9,7%

| Name der Substanz | Wirksamkeit in % | | | Synergistischer Faktor | |
|---|---|---|---|---|---|
| | Substanz allein | Substanz + Cypermethrin | Substanz + Permethrin | bei Cypermethrin | bei Permethrin |
| N-1-Pentyl-pyrrolidin | 0 | 74,8 | 100,0 | 2,37 | 10,3 |
| Pentyl-(3)-N-pyrrolidin | 0 | 84,0 | 46,8 | 2,67 | 4,82 |
| N-1-Octyl-pyrrolidin | 0 | 56,0 | 40,0 | 1,78 | 4,12 |
| N-1-(2-Ethyl)-hexylpyrrolidin | 0 | 56,0 | 41,2 | 1,78 | 4,24 |
| N-6-Undecyl-pyrrolidin | 0 | 60,0 | 58,8 | 1,90 | 6,06 |
| N-1-Tridecyl-pyrrolidin | 22,8 | 78,8 | 44,0 | 1,78 | 2,19 |
| N-1-Tetradecyl-pyrrolidin | 0 | 50,0 | 6,0 | 1,59 | – |
| 1,6-N,N-Di-pyrrolidino-hexan | 0 | 77,2 | 41,2 | 2,52 | 4,24 |
| 1,8-N,N-Di-pyrrolidino-octan | 0 | 88,0 | 64,0 | 2,79 | 6,60 |
| 1,10-N,N-Di-pyrrolidino-decan | 0 | 80,0 | 29,2 | 2,54 | 3,00 |
| cis-3-N-pyrrolidino-methylhepten-(3) | 0 | 50,8 | 16,0 | 1,61 | 1,65 |
| 3-N-pyrrolidino-3,7,11-trimethyl-1,6,10-dodecatrien | 0 | 94,8 | 34,8 | 3,00 | 3,59 |
| 3-N-pyrrolidino-propin | 0 | 50,8 | 14,8 | 1,61 | 1,52 |
| N-Cyclohexyl-pyrrolidin | 0 | 68,0 | 17,2 | 2,16 | 1,72 |
| N-Benzyl-pyrrolidin | 0 | 77,2 | 16,0 | 2,45 | 1,65 |
| N-4-Chlor-benzyl-pyrrolidin | 0 | 100,0 | 22,8 | 3,17 | 2,35 |
| 2-N-Pyrrolidino-ethanol | 0 | 81,2 | 53,2 | 2,58 | 5,48 |
| 1-N-Pyrrolidino-propandiol-(1,3) | 0 | 100,0 | 78,8 | 3,17 | 8,12 |
| N-1-(1′-Ethoxiethyl-(2))-pyrrolidin | 0 | 70,0 | 0 | 2,22 | – |
| N-1-(2′-Butoxi-diethyl-ether-(2))-pyrrolidin | 0 | 78,0 | 8,9 | 2,47 | – |
| N-1-(α-Butyl-triethylen-triether-(w))-pyrrolidin | 0 | 96,0 | 13,2 | 3,05 | 1,24 |
| 1,2-Bis-(2-N-pyrrolidino-ethoxi)-ethan | 0 | 100,0 | 69,2 | 3,17 | 7,13 |
| N-1-2-phenoxiethyl-1-N-Pyrrolidino-2-phenoxy-ethan | 0 | 53,2 | 28,0 | 1,69 | 2,88 |
| N-Cinnamyl-pyrrolidin | 0 | 97,2 | 30,8 | 3,08 | 3,17 |
| N-Piperonyl-pyrrolidin | 41,2 | 100 | 82,8 | 1,87 | 4,29 |
| Phenyl-N-pyrrolidin-essigsäure-ethylester | 0 | 92,0 | 25,2 | 2,92 | 2,60 |
| Phenyl-N-pyrrolidin-bernsteinsäure-diethylester | 0 | 88,0 | 26,8 | 2,79 | 2,76 |
| N-1-Pentyl-piperidin | 0 | 61,2 | 20,0 | 1,94 | 2,06 |
| N-1-Octyl-piperidin | 0 | 54,8 | 12,0 | 1,74 | 1,24 |
| N-1-Dodecyl-piperidin | 24,0 | 94,6 | – | 2,24 | – |
| N-1-Tridecyl-piperidin | 0 | 96,0 | 17,2 | 3,05 | 1,77 |
| 1,6-N,N-Di-piperidino-hexan | 0 | 57,2 | 2,8 | 2,82 | – |
| 1,10-N,N-Di-piperidino-decan | 0 | 76,0 | 58,8 | 2,41 | 6,06 |
| N-1-Allyl-piperidin | 0 | 73,2 | 13,2 | 2,31 | 1,36 |
| N-1-Piperidino-3,7-dimethyl-octadien-(2,6) | 0 | 88,0 | 81,2 | 2,79 | 8,37 |
| cis-8-N-Piperidino-2,6-dimethyl-octadien-(2,6) | 0 | 68,0 | 26,8 | 2,16 | 2,76 |
| N-Citronellyl-piperidin | 1,2 | 78,8 | 54,8 | 2,46 | 5,53 |
| N-Farnesyl-piperidin | 0 | 84,0 | 41,2 | 2,67 | 4,25 |
| 1-N-p-Menthen-piperidin | 0 | 60,0 | 72,0 | 1,90 | 7,42 |
| N-Benzyl-piperidin | 0 | 94,8 | 32,0 | 3,01 | 3,29 |
| N-4-Chlor-benzyl-piperidin | 0 | 74,8 | 46,8 | 2,37 | 4,82 |
| Benzal-di-N,N-piperidin | 0 | 40,0 | 18,8 | 1,26 | 1,94 |

Tabelle 3 (Fortsetzung)

| Name der Substanz | Wirksamkeit in % Substanz allein | Substanz + Cypermethrin | Substanz + Permethrin | Synergistischer Faktor bei Cypermethrin | bei Permethrin |
|---|---|---|---|---|---|
| 2-N-Piperidino-methylnaphthalin | 0 | 96,0 | 88,8 | 3,05 | 9,07 |
| N-Piperidino-pentaerythrit | 0 | 62,8 | 32,0 | 1,99 | 3,30 |
| N-1-Ethoxi-ethyl-(2)-piperidin | 0 | 56,0 | 0 | 1,78 | – |
| 2-N-Piperidino-ethyldibutylacetal | 0 | 86,8 | 36,0 | 2,76 | 3,71 |
| N-Piperidino-methyl-heptanal | 0 | 49,2 | 60,0 | 1,56 | 6,19 |
| N-2-Phenoxi-ethyl-(1)-piperidin | 4,0 | 84,0 | 68,0 | 2,54 | 6,60 |
| N-3Phenoxi-propyl-(3)-piperidin | 0 | 93,2 | 29,2 | 2,96 | 3,01 |
| N-Cinnamyl-piperidin | 0 | 97,2 | 68,0 | 3,08 | 7,01 |
| N-Piperonyl-piperidin | 0 | 90,8 | 64,0 | 2,88 | 6,60 |
| 2,5 Bis (N piperidino methyl) hydrochinon | 0 | 56,0 | 24,0 | 1,78 | 2,47 |
| 2-N-Piperdino-buttersäure-ethylester | 0 | 61,2 | 52,0 | 1,94 | 5,36 |
| N-Piperidin-bernsteinsäure-diethylester | 0 | 97,2 | 24,0 | 3,08 | 2,47 |
| Phenyl-N-piperidino-essigsäure-benzylester | 0 | 96,0 | 28,0 | 3,05 | 2,89 |
| N-Dimethyl-carbamoyl-piperidin | 0 | 53,2 | 64,0 | 1,69 | 6,60 |
| N-1-Dodecyl-ethylenimin | 0 | 90,0 | 36,0 | 2,86 | 3,71 |
| N-1-Linalyl-ethylenimin | 0 | 97,2 | 65,2 | 2,99 | 6,72 |
| N-1-Nonyl-hexamethylenimin | 40,0 | 97,2 | 84,0 | 1,82 | 4,54 |
| N-1-Undecyl-hexamethylenimin | 0 | 73,1 | 81,2 | 2,32 | 8,37 |
| N-1-Dodecyl-hexamethylenimin | 14,0 | 64,0 | 24,0 | 1,59 | 1,03 |
| 6-N-Hexamethylenimino-2,6-dimethyl-octadien-(2,7) | 0 | 76,0 | 70,8 | 2,41 | 7,30 |
| N-(2'-Butoxi-diethylether-(2))-hexamethylenimin | 0 | 78,8 | 32,0 | 2,50 | 3,30 |
| N-2-Phenoxi-ethyl-(1))-hexamethylenimin | 0 | 85,2 | 49,4 | 2,70 | 5,09 |
| 1-N-Hexamethylenimino-3-phenoxi-propan | 0 | 64,0 | 33,2 | 2,03 | 3,42 |
| N-Piperonyl-hexamethylenimin | 0 | 96,0 | 68,0 | 3,05 | 7,01 |
| N-Dimethyl-carbamoyl-hexamethylenimin | 0 | 77,2 | 53,2 | 2,45 | 5,48 |
| N-1-Dodecal-2-methylpiperidin | 0 | 46,8 | 20,0 | 1,49 | 2,06 |
| N-1-Octyl-2-ethyl-piperidin | 26,8 | 74,8 | 52,0 | 1,52 | 2,60 |
| N-1-Hexadecyl-2-ethylpiperidin | 0 | 74,8 | 18,8 | 2,37 | 1,94 |
| N-1-Propanrgyl-2-ethyl-piperidin | 0 | 92,0 | 37,2 | 2,92 | 3,84 |
| N-Benzyl-2-ethyl-piperidin | 0 | 78,8 | 14,8 | 2,50 | 1,53 |
| N-1-Pentyl-morpholin | 0 | 88,0 | 37,2 | 2,79 | 3,83 |
| N-1-Decyl-morpholin | 0 | 73,1 | 81,2 | 2,32 | 8,37 |
| N-1-Dodecyl-morpholin | 0 | 86,8 | 14,8 | 2,76 | 1,53 |
| N-1-Tetradecyl-morpholin | 0 | 64,0 | 5,2 | 2,03 | – |
| N-Cycloheptyl-morpholin | 0 | 56,0 | 34,8 | 1,78 | 3,59 |
| N-Linalyl-morpholin | 0 | 92,0 | 33,2 | 2,92 | 3,42 |
| N-Benzyl-morpholin | 0 | 45,2 | 21,2 | 1,43 | 2,19 |
| N-1-Phenyl-ethyl-(2)-morpholin | 0 | 86,8 | 73,2 | 2,76 | 7,55 |
| N-Diphenyl-methyl-morpholin | 0 | 72,0 | 40,0 | 2,29 | 4,12 |
| N-Ethanol-morpholin | 0 | 66,8 | 38,8 | 2,12 | 4,0 |
| N-(2'-Butoxi-diethyl-ether-(2))-morpholin | 0 | 93,2 | 78,8 | 2,96 | 8,12 |

Tabelle 3 (Fortsetzung)

| Name der Substanz | Wirksamkeit in % Substanz allein | Substanz + Cypermethrin | Substanz + Permethrin | Synergistischer Faktor bei Cypermethrin | bei Permethrin |
|---|---|---|---|---|---|
| 2-N-Morpholino-ethyl-dibutyl-acetal | 0 | 100 | 100 | 3,17 | 10,31 |
| N-(2-Benzyloxi-ethyl-(1))-morpholin | 0 | 50,8 | 32,0 | 1,61 | 3,30 |
| N-(2-Phenoxi-ethyl-(1))-morpholin | 0 | 77,2 | 62,8 | 2,45 | 6,47 |
| N-Cinnamyl-morpholin | 0 | 54,8 | 44,0 | 1,74 | 4,54 |
| N-Piperonyl-morpholin | 0 | 73,2 | 37,2 | 2,32 | 3,84 |
| N-Morpholino-propionsäure-ethylester | 0 | 65,2 | 26,8 | 2,07 | 2,76 |
| 2-N-Morpholino-buttersäure-ethylester | 0 | 49,2 | 32,0 | 1,56 | 3,30 |

Beispiel 4

Die synergistische Wirkung der tertiären, heterocyclischen Amine auf die Pyrethroide ist auch dann noch deutlich, wenn das Amin in wesentlich niederen Aufwandmengen eingesetzt wird, als dies in Beispiel 3 beschrieben wurde. Die Ergebnisse sind in der Tabelle 4 zusammengestellt. Dieser Versuch zeigt auch, dass Amine mit insektiziden Eigenschaften, wenn sie in Konzentrationen unterhalb ihres insektiziden Wirkungsbereiches eingesetzt werden, immer noch gute synergistische Wirkungen im Gemisch mit Pyrethroiden zeigen. Die Versuche wurden wie in Beispiel 3 beschrieben mit Kornkäfern durchgeführt. Es wurden lediglich die Konzentrationen der eingesetzten tertiären, heterocyclischen Amine geändert. Für die Verdünnung Pyrethroid : Amin = 1 : 10 wurde das Amin mit 0,1 mg Wirkstoff/ml Acetonlösung eingesetzt. Für die Verdünnung 1 : 5 mit 0,05 mg Wirkstoff/ml.

Tabelle 4

Synergistische Wirkung von tertiären heterocyclischen Aminen auf Pyrethroide bei niederen Aufwandmengen des Amins als in Tabelle 3, getestet an sitophilus granarius L.

Wirksamkeit von Cypermethrin: 31,5%

Wirksamkeit von Permethrin: 9,7%

| Name der Substanz | Verhältnis Pyrethroid zu Amin | % Wirkung Substanz allein | Substanz + Cypermethrin | Substanz + Permethrin | Synergistischer Faktor bei Cypermethrin | bei Permethrin |
|---|---|---|---|---|---|---|
| N-1-Undecyl-pyrrolidin | 1:10 | 0 | 97,2 | 72,0 | 3,09 | 7,42 |
| N-1-4-Chlor-benzylpyrrolidin | 1:10 | 0 | 97,2 | 73,2 | 3,09 | 7,55 |
| N-1-Decyl-piperidin | 1:10 | 0 | 97,2 | 65,2 | 3,09 | 6,72 |
| N-1-Undecyl-piperidin | 1: 5 | 0 | 90,8 | 53,2 | 2,88 | 5,48 |
| N-1-Undecyl-piperidin | 1:10 | 0 | 97,2 | 60,0 | 3,09 | 6,19 |
| N-Piperonyl-piperidin | 1: 5 | 0 | 84,0 | 54,8 | 2,67 | 5,65 |
| N-Piperonyl-piperidin | 1:10 | 0 | 74,8 | 24,0 | 2,34 | 2,47 |
| N-Benzyl-piperidin | 1:10 | 0 | 100,0 | 73,2 | 3,17 | 7,55 |
| Phenyl-N-piperidino-essigsäure-ethylester | 1:10 | 0 | 98,8 | 50,8 | 3,14 | 5,24 |
| N-1-Undecyl-hexamethylenimin | 1:10 | 0 | 92,0 | 73,2 | 2,92 | 7,55 |

Beispiel 5

Die erfindungsgemässen Verbindungen haben nur eine geringe Toxizität. Es wurde die akute, orale Toxizität von Phenyl-N-piperidino-essigsäure-ethylester und 1-N-Undecyl-piperidin bestimmt. Die Tests wurden mit SPF-Wistar-Ratten durchgeführt und die Wirkstoffe einmal oral appliziert.

Die 14 Tage $LD_{50}$ von Phenyl-N-piperidino-essigsäure-ethylester liegt bei 9751,3 mg/kg bei männlichen Ratten und bei 8174,8 mg/kg bei weiblichen Ratten.

Die 14 Tage $LD_{50}$ von 1-N-Undecyl-piperidin liegt bei 592,7 mg/kg bei männlichen Ratten und bei 492,6 mg/kg bei weiblichen Ratten.

Beispiel 6

Herstellung von N-Linalyl-ethylenimin (6-N-Aziridino-2,6-dimethyl-octadien-(2,7))

17,3 g Linalylchlorid wurden mit 4,3 g Aziridin versetzt. Die Mischung wurde in einem kleinen Kolben am Rückflusskühler mit Natronkalkrohr auf dem Infrarotheizbad zum gelinden Sieden erhitzt. Jede Stunde wurde eine Probe entnommen und argentometrisch titriert. Nach 3 h wurde die Reaktion beendet, die Umsetzung war zu 97,89% gelaufen. Die Mischung wurde abgekühlt und im Scheidetrichter mit der Lösung von 5,6 g Kaliumhydroxid in 50 ml Wasser ausgeschüttelt. Die organische Phase wurde im Ölpumpenvakuum fraktioniert. Es wurden 7,0 g einer farblosen Flüssigkeit erhalten, die bei 94–99° bei $7{,}98 \cdot 10^{-4}$ bar destillierte. Eine acidimetrische Titration ergab ein Äquivalentgewicht von 176,0, es berechnet sich ein solches von 179,3.

Beispiel 7

Herstellung von N-1-Undecyl-hexamethylenimin

23,5 g 1-Brom-undecan wurden mit 75 ml Methylenchlorid versetzt. Die Mischung wurde unter mechanischem Rühren am Rückflusskühler mit Natronkalkrohr im Wasserbad zum Sieden erhitzt. Zu der siedenden Lösung wurde innerhalb von 20 min die Lösung von 19,8 g Hexamethylenimin in 25 ml Methylenchlorid getropft. Die Mischung wurde noch 30 min lang weiter erhitzt, dann wurde abgekühlt. Vom Niederschlag wurde abgesaugt. Das Filtrat wurde im Scheidetrichter mit 25 ml Wasser ausgeschüttelt. Aus der organischen Phase wurde das Methylenchlorid abdestilliert. Der Rückstand wurde im Ölpumpenvakuum fraktioniert. Es wurden 9,5 g einer farblosen Flüssigkeit erhalten, die bei 135°C bei $2{,}66 \cdot 10^{-3}$ bar destillierte. Aus einer acidimetrischen Titration errechnete sich ein Äquivalentgewicht von 254,6, es berechnet sich ein solches von 253,5.

Beispiel 8

Herstellung von 1-N-Piperidino-3,7-dimethyl-octadien-(2,6)

Ein Gemisch von 8,6 g 1-Chlor-3,7-dimethyl-octadien-(2,6) (Geranylchlorid) und 10 ml Piperidin wurde in einem kleinen Kolben am Rückflusskühler mit Natronkalkrohr auf dem Infrarotheizbad zum gelinden Sieden erhitzt. Nach jeweils einer Stunde wurde eine Probe entnommen und argentometrisch titriert. Nach 4 Stunden war die Umsetzung zu 98,6% erfolgt. Die Mischung wurde abgekühlt und im Scheidetrichter mit 25 ml Wasser ausgeschüttelt. Die organische Phase wurde im Ölpumpenvakuum fraktioniert destilliert. Es wurden 6,2 g einer schwach gelblich gefärbten Flüssigkeit erhalten, die bei 120°C bei $2{,}66 \cdot 10^{-3}$ bar destillierte. Bei einer Stickstoffbestimmung nach Kjeldahl wurde 6,26% N gefunden (Theorie: N = 6,33%).

Beispiel 9

Herstellung von N-Dimethyl-carbamoyl-hexamethylenimin

10,7 g frisch destilliertes Dimethylcarbamoylchlorid wurden in 50 ml Methylenchlorid gelöst. Dazu wurde unter mechanischem Rühren am Rückflusskühler mit Natronkalkrohr und Kühlen mit fliessendem Wasser im Verlauf von 20 min die Lösung von 19,8 g Hexamethylenimin in 35 ml Methylenchlorid zugetropft. Die Mischung wurde anschliessend noch eine Stunde lang weiter gerührt. Es wurde vom Niederschlag bei schwachem Vakuum abgesaugt. Aus dem Filtrat wurde das Methylenchlorid abdestilliert und der Rückstand im Ölpumpenvakuum fraktioniert. Es wurden 10,0 g einer farblosen Flüssigkeit erhalten, die bei 95°C bei $1{,}48 \cdot 10^{-3}$ bar destillierte. Eine Stickstoffbestimmung nach Kjeldahl ergab einen Stickstoffgehalt von 16,35% (Theorie: N = 16,46%).

Beispiel 10

Herstellung von 6-N-Hexamethylenimino-2,6-dimethyl-octadien-(2,7)

10 g Linalylchlorid wurden mit 5,8 g Hexamethylenimin versetzt. Die Mischung wurde in einem kleinen Kolben am Rückflusskühler mit Natronkalkrohr auf dem Infrarotheizbad zum gelinden Sieden erhitzt. Stündlich wurde eine Probe entnommen und argentometrisch titriert. Nach 6 h wurde die berechnete Menge an Chlorid gefunden. Die Mischung wurde abgekühlt und mit der Lösung von 3,2 g Kaliumhydroxid in 30 ml Wasser im Scheidetrichter ausgeschüttelt. Die organische Phase wurde noch einmal mit 20 ml Wasser ausgeschüttelt. Dann wurde sie im Ölpumpenvakuum fraktioniert. Es wurden 5,3 g einer farblosen Flüssigkeit erhalten, die bei 136–140°C bei $1{,}33\ 10^{-3}$ bar destillierte. Bei einer acidimetrischen Titration wurde ein Äquivalentgewicht von 230,7 gefunden (Theorie 235,4).

**Patentansprüche**

1. Verwendung tertiärer, heterocyclischer Amine der allgemeinen Formel (I)

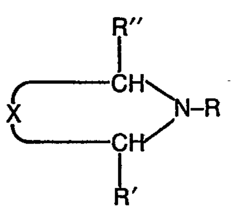

I

worin

R ein aliphatischer Rest mit 1 bis 22 Kohlenstoffatomen, der durch Aryl-, Hydroxyl-, Alkoxy- und/oder Carboxylestergruppen oder durch den Rest des heterocyclischen Amins der Formel (I) (ohne R) substituiert sein kann und der ausserdem durch Äther- oder Estergruppen unterbrochen sein kann, oder Piperonyl

R′ und R″ unabhängig voneinander jeweils H, $CH_3$ oder $C_2H_5$, und

X die Gruppe $-(CH_2)-_n$, worin n eine ganze Zahl von 0 bis 5 ist, oder die Gruppe $-CH_2-O-CH_2-$ bedeuten, als Potenzierungsmittel für insektizide Pyrethroide und/oder Carbamate und/oder Phosphorsäurederivate.

2. Verwendung von N-4-Chlorbenzylpyrrolidin oder -piperidin als Potenzierungsmittel für insektizide Pyrethroide.

3. Verwendung von N-Piperonyl-piperidin, -morpholin, -pyrrolidin oder -hexamethylenimin für die Zwecke des Anspruchs 1.

4. Verwendung von 2,5-Bis-(N-piperidino-methyl)-hydrochinon für die Zwecke des Anspruchs 1.

5. Verwendung von N-Piperidino-methyl-heptanal als Potenzierungsmittel für insektizide Pyrethroide.

6. Verwendung von Dimethylcarbamoylpiperidin oder -hexamethylenimin als Potenzierungsmittel für insektizide Pyrethroide.

7. Verwendung von N-1-Pentyl-pyrrolidin als Potenzierungsmittel für Permethrin.

8. Verwendung von 1-N-Pyrrolidinopropandiol-(1,3) als Potenzierungsmittel für Permethrin.

9. Verwendung von 1-N-Piperidino-3,7-dimethyl-octadien-(2,6) als Potenzierungsmittel für Permethrin.

10. Verwendung von N-Cinnamyl-pyrrolidin als Potenzierungsmittel für Dimetilan.

11. Insektizides Gemisch auf der Basis von insektiziden Pyrethroiden, Carbamaten oder Phosphorsäurederivaten, dadurch gekennzeichnet, dass es die tertiären heterocyclischen Amine der allgemeinen Formel I des Patentanspruchs 1 in einem Mengenverhältnis zum eingesetzten Insektizid von 0,2 : 1 bis 100 : 1 enthält.

12. N-Linalyl-ethylenimin.

13. N-1-Undecyl-hexamethylenimin.

14. N-Dimethyl-carbamoyl-hexamethylenimin.

15. 6-N-Hexamethylenimino-2,6-dimethyl-octadien-(2,7).

**Claims**

1. The use of tertiary, heterocyclic amines of the general formula (I)

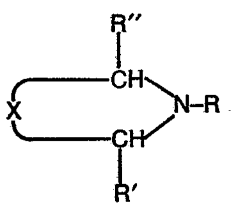

I

in which

R is an aliphatic radical with 1 to 22 carbon atoms, which may be substituted by aryl-, hydroxyl-, alkoxy, and/or carboxylester groups or by the radical of the heterocyclic amine of Formula I (without R) and which in addition may be interrupted by ether- or ester groups, or piperonyl

R′ and R″ independently of each other are respectively H, $CH_3$ or $C_2H_5$, and

X is the group $-(CH_2)_n-$, in which n is an integer from 0 to 5, or the group $-CH_2-O-CH_2-$, as potentiating agent for insecticidal pyrethroids and/or carbamates and/or phosphoric acid derivatives.

2. The use of N-4-chlorobenzylpyrrolidine or -piperidine as potentiating agent for insecticidal pyrethroids.

3. The use of N-piperonyl-piperidine, -morpholine, -pyrrolidine or -hexamethylene-imine for the purposes of Claim 1.

4. The use of 2,5-bis-(N-piperidino-methyl)-hydroquinone for the purposes of Claim 1.

5. The use of N-piperidino-methyl-heptanal as potentiating agent for insecticidal pyrethroids.

6. The use of dimethylcarbamoylpiperidine or -hexamethyleneimine as potentiating agent for insecticidal pyrethroids.

7. The use of N-1-pentyl-pyrrolidine as potentiating agent for permethrin.

8. The use of 1-N-pyrrolidinopropanediol-(1,3) as potentiating agent for permethrin.

9. The use of 1-N-piperidino-3,7-dimethyl-octadiene-(2,6) as potentiating agent for permethrin.

10. The use of N-cinnamyl-pyrrolidine as potentiating agent for dimetilan.

11. Insecticidal mixture based on insecticidal pyrethroids, carbamates or phosphoric acid derivatives, characterised in that it contains the tertiary heterocyclic amines of the general formula I of Patent Claim 1 in a quantitative ratio to the insecticide employed of 0.2 : 1 to 100 : 1.

12. N-Linalyl-ethyleneimine.

13. N-1-Undecyl-hexamethyleneimine.

14. N-Dimethyl-carbamoyl-hexamethyleneimine.

15. 6-N-Hexamethyleneimino-2,6-dimethylocta-diene-(2,7).

**Revendications**

1. Utilisation d'amines hétérocycliques tertiaires de formule générale (I)

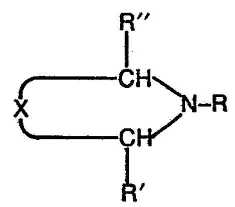

I

dans laquelle

R représente un reste aliphatique en $C_1$–$C_{22}$ qui peut être substitué par des groupes aryle, hydroxy, alcoxy et/ou ester carboxylique ou par le reste de l'amine hétérocyclique de formule I (sans R) et peut en outre être interrompu par des groupes éther ou ester, ou un groupe pipéronyle,

R′ et R″ représentent chacun, indépendamment l'un de l'autre H, $CH_3$ ou $C_2H_5$, et

X représente le groupe $-(CH_2)_n-$, dans lequel n est un nombre entier de 0 à 5, ou le groupe $-CH_2-O-CH_2-$,

en tant qu'agents potentialisants pour des pyréthroïdes et/ou des carbamates et/ou des dérivés de l'acide phosphorique insecticides.

2. Utilisation de la N-4-chlorobenzylpyrrolidine ou -pipéridine en tant qu'agent potentialisant pour des pyréthroïdes insecticides.

3. Utilisation de la N-pipéronyl-pipéridine, -morpholine, -pyrrolidine ou -hexaméthylène-imine pour les buts de la revendication 1.

4. Utilisation de la 2,5-bis-(N-pipéridino-méthyl)-hydroquinone pour les buts de la revendication 1.

5. Utilisation du N-pipéridino-méthyl-heptanal en tant qu'agent potentialisant pour des pyréthroïdes insecticides.

6. Utilisation de la diméthylcarbamoylpipéridine ou -hexaméthylène-imine en tant qu'agent potentialisant pour des pyréthroïdes insecticides.

7. Utilisation de la N-1-pentyl-pyrrolidine en tant qu'agent potentialisant pour le Permethrin.

8. Utilisation du 1-N-pyrrolidinopropane-diol-(1,3) en tant qu'agent potentialisant pour le Permethrin.

9. Utilisation du 1-N-pipéridino-3,7-diméthyl-octadiène-(2,6) en tant qu'agent potentialisant pour le Permethrin.

10. Utilisation de la N-cinnamyl-pyrrolidine en tant qu'agent potentialisant pour le Dimetilan.

11. Mélange insecticide à base de pyréthroïdes, carbamates ou dérivés de l'acide phosphorique insecticides, caractérisé en ce qu'il contient des amines hétérocycliques tertiaires de formule générale I de la revendication 1 en proportion relative de 0,2 : 1 à 100 : 1 avec l'insecticide mis en œuvre.

12. La N-linalyl-éthylène-imine.

13. La N-1-undécyl-hexaméthylène-imine.

14. La N-diméthyl-carbamoyl-hexaméthylène-imine.

15. Le 6-N-hexaméthylène-imino-2,6-diméthyl-octadiène-(2,7).